# EUROPÄISCHE PATENTSCHRIFT

(11) **EP 1 305 058 B1**
(45) Veröffentlichungstag und Bekanntmachung des Hinweises auf die Patenterteilung: **25.05.2005**
(21) Anmeldenummer: 01962585.4
(22) Anmeldetag: 01.08.2001
(51) Int. Cl.: A61L 27/22, A61L 27/54

(54) **VERFAHREN ZUR HERSTELLUNG BIOAKTIVER IMPLANTATOBERFLÄCHEN**
METHOD FOR PRODUCING BIOACTIVE IMPLANT SURFACES
PROCEDE DE PRODUCTION DE SURFACES D'IMPLANTS BIOACTIVES

(30) Priorität: 01.08.2000 DE 10037850
(43) Veröffentlichungstag der Anmeldung: 02.05.2003
(73) Patentinhaber: Morphoplant GmbH, 44801 Bochum (DE)
(72) Erfinder: JENNISSEN, Herbert, P., 50858 Köln (DE); CHATZINIKOLAIDOU, Maria, 45147 Essen (DE); RUMPF, Heike, 45770 Marl (DE)
(74) Vertreter: Viering, Jentschura & Partner
(86) Internationale Anmeldenummer: PCT/DE2001/002893
(87) Internationale Veröffentlichungsnummer: WO 2002/009788

(56) Entgegenhaltungen:
- WO-A-00/25841
- WO-A-92/00047
- WO-A-99/26674
- US-A- 4 652 459
- US-A- 4 828 563

## Beschreibung

Die vorliegende Erfindung betrifft ein Verfahren zur Herstellung bioaktiver Implantatoberflächen von metallischen oder keramischen Materialien, die für Implantate wie künstliche Gelenke, Zahnimplantate oder auch Kleinstimplantate, z.B. sogenannte Stents, verwendet werden, als auch nach dem Verfahren hergestellte Implantate, die als sogenannte "aktive device" eine kontrollierte Freisetzung der bioaktiven Moleküle von den Implantatmaterialen erlauben.

Die Implantation künstlicher Gelenke oder Knochen hat in den letzen Jahren eine zunehmende Bedeutung z.B. bei der Behandlung von Gelenkdysplasien oder -luxationen bzw. bei Erkrankungen, die auf der Abnutzung von Gelenken als Folge von Gelenkfehlstellungen entstehen können, gewonnen. Die Funktion der Implantate und die für ihre Herstellung verwendeten Materialien, die neben Metallen wie Titan oder Metallegierungen auch Keramik oder Kunststoffmaterialien wie Teflon umfassen können, sind stetig verbessert worden, so dass Implantate nach erfolgreichem Heilungsverlauf in 90-95% der Fälle Standzeiten von 10 Jahren aufweisen können. Ungeachtet dieser Fortschritte und verbesserter operativer Verfahren bleibt eine Implantation immer noch ein schwieriger und belastender Eingriff, insbesondere da sie mit einem langwierigen Einheilungsprozeß des Implantates, der oft monatelange Klinik- und Kuraufenthalte einschließlich Rehabilitationsmaßnahmen umfaßt, verbunden ist. Neben den Schmerzen stellen dabei für die betroffenen Patienten die Länge der Behandlungsdauer und die Trennung von der vertrauten Umgebung große Belastungen dar. Ferner verursacht der langwierige Heilungsprozeß durch die erforderliche intensive Betreuung hohe Personal- und Pflegekosten.

Das Verständnis der Vorgänge auf der molekularen Ebene, die für ein erfolgreiche Einwachsen eines Implantates erforderlich sind, hat sich in den letzten Jahren bedeutend erweitert. Entscheidend für die Gewebeverträglichkeit eines Implantates sind Strukturkompatibilität und Oberflächenkompatibilität. Die Biokompatibilität im engeren Sinne ist alleine von der Oberfläche bedingt. Auf allen Ebenen der Integration spielen Proteine eine maßgebliche Rolle. Wie nachfolgend erläutert, entscheiden sie bereits während der Implantationsoperation durch die Ausbildung einer initialen adsorbierten Proteinschicht über den weiteren Verlauf der Implantateinheilung, da sich auf dieser Schicht später die ersten Zellen ansiedeln.

Bei der molekularen Interaktion zwischen Implantat, das auch als Biomaterial bezeichnet wird, und Gewebe, findet eine Vielzahl von Reaktionen statt, die streng hierarchisch geordnet zu sein scheinen. Als erste biologische Reaktion findet die Adsorption von Proteinen an der Oberfläche des Biomaterials statt. In der dadurch entstandenen Proteinschicht werden anschließend einzelne Proteinmoleküle beispielsweise entweder durch Konformationsänderungen zu Signalstoffen umgewandelt, die auf der Oberfläche präsentiert werden, oder durch katalytische (proteolytische) Reaktionen werden als Signalstoffe wirkende Proteinfragmente freigesetzt. Ausgelöst durch die Signalstoffe, findet in der nächsten Phase die zelluläre Besiedlung statt, die eine Vielzahl von Zellen wie Leukozyten, Makrophagen, Immunozyten, und schließlich auch Gewebezellen (Fibroblasten, Fibrozysten, Osteoblasten, Osteozyten) umfassen kann. In dieser Phase spielen andere Signalstoffe, sogenannte Mediatoren, wie z.B. Cytokine, Chemokine, Morphogene, Gewebshormone und echte Hormone eine entscheidende Rolle. Im Falle einer Biokompatibilität kommt es schließlich zur Integration des Implantates in den Gesamtorganismus, und idealerweise erhält man ein Permanentimplantat.

Im Licht von Arbeiten, die in den letzten Jahren auf molekularer Ebene der Osteogenese durchgeführt worden sind, haben chemische Signalstoffe, die sogenannten "bone morphogenic Proteins" (BMP-1-BMP-14), die Einfluß auf das Knochenwachstum besitzen, eine zunehmende Bedeutung gewonnen. BMPs (insbesondere BMP-2 und BMP-4, BMP-5, BMP-6, BMP-7) sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von alkalischer Phosphatase, Hormonrezeptoren, knochenspezifischer Substanzen wie Kollagen Typ 1, Osteocalcin, Osteopontin und schließlich die Mineralisation. Die BMP-Moleküle regulieren dabei die drei Schlüsselreaktionen Chemotaxis, Mitose und Differenzierung der jeweiligen Vorläuferzelle. Darüber hinaus spielen BMPs eine wichtige Rolle in der Embryogenese, Organogenese des Knochens und anderer Gewebe, wobei als Zielzellen Osteoblasten, Chondroblasten, Myoblasten und vaskulare glatte Muskelzellen (Proliferationshemmung durch BMP-2) bekannt ist.

Inzwischen sind 14 BMPs inklusive multipler Isoformen bekannt. Bis auf das BMP-1 gehören die BMPs der "transforming growth factor beta" (TGF-β) Superfamilie an, für die spezifische Rezeptoren auf den Oberflächen der entsprechenden Zellen nachgewiesen wurden. Wie der erfolgreiche Einsatz von rekombinanten humanen BMP-2 und/oder BMP-7 in Versuchen zu Defektheilungsprozessen an Ratten, Hunden, Kaninchen und Affen gezeigt hat, scheint keine Speziesspezifität vorzuliegen. Bisherige Versuche, die knochenbildungsauslösenden Eigenschaften der BMPs gezielt für Implantationszwecke auszunutzen, indem das BMP-2 und/oder BMP-7 nicht-kovalent auf metallische oder keramische Biomaterialien aufgebracht wurden, sind jedoch weitestgehend erfolglos verlaufen.

Die Aufgabe der vorliegenden Erfindung besteht darin, verbesserte Biomaterialien zur Verwendung als Implantate bereitzustellen, die sich durch eine erhöhte Beladungsdichte mit Mediatormolekülen, insbesondere BMPs, und eine verlängerte Langzeitabgabe in das die Implantate umgebende Gewebe auszeichnen.

Erfindungsgemäß wird diese Aufgabe dadurch gelöst, dass ein Verfahren zur Herstellung bioaktiver Implantatoberflächen von metallischen oder keramischen Materialien bereitgestellt wird, bei dem in einem ersten Schritt Ankermoleküle mit hydrophoben Resten an der Oberfläche des Implantatmaterials kovalent gebunden werden und in einem zweiten Schritt Mediatormoleküle auf das so behandelte Implantatmaterial gegeben werden, die infolge nicht-kovalenter Wechselwirkungen zwischen den Mediatormolekülen und den hydrophoben Resten der Ankermoleküle immobilisiert werden, wobei im ersten Schritt die Beladungsdichte der Ankermoleküle auf der Implantatoberfläche in Abhängigkeit von der Kettenlänge des hydrophoben Restes des Ankermoleküls so gewählt wird, daß die Ankermoleküle selbst miteinander nicht in Wechselwirkung treten und in Abhängigkeit von der überdeckten Fläche auf dem Implantatmaterial, die von einem einzelnen im zweiten Schritt absorbierten Mediatormolekül bedeckt wird, mindestens 10, bevorzugt 15 Kontaktstellen zwischen den hydrophoben Resten der Ankermoleküle zur hydrophoben Wechselwirkung mit dem Mediatormolekül gebildet werden.

Unter der nicht gewünschten Wechselwirkung zwischen den Ankermolekülen ist in erster Linie eine sterische Wechselwirkung zu verstehen, die hier nicht gewünscht ist, damit die Ankermoleküle sterisch voneinander ungehindert mit den Mediatormolekülen in Wechselwirkung treten können.

Unter Kontaktstelle ist erfindungsgemäß der Ort der größten hydrophoben Wechselwirkung zwischen einem Rest der Ankermoleküle und dem Mediatormolekül zu verstehen. Dabei können an einem Rest durch Verzweigung des Restes mehrere Kontaktstellen vorhanden sein. So kann eine endständig mit einer Methylgruppe substituierte Kohlenstoffkette zumindest zwei Kontaktstellen besitzen. Die Erfinder haben erkannt, daß es bei der Immobilisierung der Mediatormoleküle durch hydrophobe Wechselwirkung entscheidend auf die Anzahl der Kontaktstellen zur hydrophoben Wechselwirkung zwischen den Resten und dem Mediatormolekül ankommt. Dabei sind möglichst nah benachbarte Kontaktstellen von Vorteil, so daß stärker verzweigte Reste bevorzugt sind, da hierbei mehrere benachbarte Kontaktstellen zur Verfügung stehen. Beispielsweise ist eine endständige Trimethylgruppe an einem hydrophoben Rest gegenüber einer geradkettigen unverzweigten Kette mit gleicher Gesamtzahl an Kohlenstoffatomen bevorzugt.

Bei dem erfindungsgemäßen Immobilisierungsverfahren wird insbesondere ein Substitutionsgrad des Ankermoleküls, damit indirekt (d.h. Oberflächenkonzentration des immobilisierten Proteins), erreicht, der eine multivalente Wechselwirkung zwischen Oberfläche und Zelle erlaubt und ermöglicht, die Knochen- oder Gewebsbildung wirksam zu steuern.

Bei dem erfindungsgemäßen Verfahren werden bevorzugt in einem ersten Schritt Alkyl-, Alkenyl- oder Alkinyl- oder Arylreste mit 1 bis 30, bevorzugt 3 bis 20, besonders bevorzugt 3 bis 8 Kohlenstoffatome, die auch durch Silizium in der Alkylkette und/oder Heteroatome wie N, O oder S in der Alkylkette und/oder im Arylring ersetzt sein können, bevorzugt in einer verzweigten Kette, die auch gegebenenfalls mit einem oder mehreren Substituenten von Halogen, Alkoxy-, Hydroxyl-, Thiol-, Amino-, Alkyl- oder Dialkylamino-Gruppen, wobei die Alkylgruppen des Substituenten bevorzugt 1 - 6 Kohlenstoffatome aufweisen und geradkettig oder verzweigt sein können, substituiert sein können, vorzugsweise aber unsubstituiert und besonders bevorzugt verzeigt sind, an die Oberfläche des Implantatmaterials kovalent gebunden. Diese Bindung der Reste kann jeweils über eine Kopplung über eine Silylgruppe, eine Bromcyan-Gruppe oder eine Aminogruppe, beispielsweise eines Aminoalkans, erfolgen.

In einem zweiten Schritt werden die biologischaktiven Knochenwachstumsfaktoren als Mediatormoleküle mittels nicht-kovalenter Bindung, vermutlich aufgrund hydrophober Wechselwirkungen auf dem Implantatmaterial immobilisiert werden. Dadurch wird ermöglicht, eine chemotaktisch wirkende und/oder biologisch aktive, eine sogenannte juxtakrine, Implantatoberfläche auszubilden, die zur Ansiedlung, Proliferation und Ausdifferenzierung von Knochenzellen führt. So lassen sich sogenannte aktive Implantate bereitstellen, die bei von der Oberfläche freigesetzten Molekülen auch auf eine Entfernung von 500 bis 1000 im eine chemotaktische Wirkung auf Zellen, im Falle von BMPs auf Osteoblasten, zeigen.

Die Bestimmung der Beladungsdichte der Implantatoberfläche mit Ankermolekülen, die in der Regel nur einen hydrophoben Rest mit, je nach Verzweigungsgrad, mindestens einer Kontaktstelle besitzen, erfolgt in der Regel ausgehend von der Größenabschätzung des gewöhnlich als Ellipsoid vorliegenden Mediatormoleküls. Danach wird nach Kenntnis der auf die Implantatoberfläche projizierten Fläche des Mediatormoleküls die Anzahl der erforderlichen Kontaktstellen mit mindestens 10 bestimmt und in Abhängigkeit davon die Kettenlänge und der Verzweigungsgrad der Ankermoleküle festgelegt. Daraus errechnet sich dann die Beladungsdichte.

Anfängliche Untersuchungen der Erfinder ergaben, daß nach Modifikation von Titanoberflächen mit Aminopropylsilan (APS) die Anzahl der immobilisierten Aminogruppen mit dem Bolton-Hunter Reagens bestimmt Werte im Bereich von 1.0-2.5 nmol/cm² ergaben. Unter Berücksichtigung der Loschmidt'schen Zahl ergeben sich bei 1 nmol/cm² ca. 60 Moleküle/10 nm². Aus diesem Wert kann man einen mittleren Abstand der APS-Moleküle von einander von ca. 0.4-0.5 nm errechnen, was als vernünftiger Wert erscheint.

Im Falle der Kopplung des Proteins Ubiquitin (m = 8.5 kDa) erhielten die Erfinder Maximalwerte von 1-2 µg/cm². Bei Rechnung mit 1 µg/cm² erhält man 3.85 x 10-11 Mole/cm². Die Umrechnung auf Moleküle ergibt dann 2.3 Moleküle Ubiquitin pro 10 nm², somit einen mittleren Abstand der Moleküle unter der Annahme einer punktförmigen Größe von 6-7 nm, was bei einer geschätzten tatsächlichen Größe von 3-4 nm Durchmesser für das Ubiquitin-Molekül eine recht hohe Packungsdichte in Form vermutlich einer Monoschicht auf der Oberfläche bedeutet. Da man bei der Adsorption von Ubiquitin ähnlich hohe Werte (wie bei der Kopplung von BMP-2) im Bereich von 1-3 µg/cm² (= 2-6 Mole Ubiquitin/10 nm²) erhält, konnten die Erfinder berechnen, daß im Schnitt einem Molekül Ubiquitin 10-30 APS Moleküle APS (60/6 und 60/2) für eine Wechselwirkungsreaktion zur Verfügung stehen. Somit konnten die Erfinder abschätzen, daß ein Molekül Ubiquitin eine Fläche bedeckt (sog. "Footprint"), das in etwa diese Anzahl von APS-Molekülen enthält, d.h. es können maximal 10-30 APS-Moleküle theoretisch mit einem Molekül Ubiquitin reagieren, wobei von einer statistischen Reaktion auszugehen ist.

Unter der Annahme einer hydrophoben Adsorption werden nach Feststellung der Erfinder nicht alle (d.h. 30) Propylreste mit dem Ubiquitin reagieren können, da es nicht so viele "hydrophobic patches" für eine geometrisch definierte Bindung auf einer Seite des Moleküls besitzt. Nach Schätzung der Erfinder werden daher maximal 4-10 Alkylreste am Ubiquitin eine spezifische Bindungsstelle finden können und tatsächlich zur Adsorption von Ubiquitin führen.

Reduziert man nun den Substitutionsgrad d.h. die Anzahl der Alkylreste/10 nm², wird der Abstand so groß, daß nicht mehr genügend Reste mit dem Ubiquitin reagieren können, und es findet keine Adsorption mehr statt. Anderseits, wenn man die Alkylkettenlänge erhöht, wird die Bindungsenergie einer Alkylkette mit dem Protein erhöht, und man benötigt nur noch weniger Alkylreste, um ein Molekül Ubiquitin zu binden.

Bei Verwendung von BMP-2 (m = 26 kDa) mit einer Größe von ca. 4 x 4 x 8 nm besitzen (Bindung auf Längsseite) haben die Erfinder zunächst eine maximale Belegung von etwa 0.5 - 1 Molekül pro 10nm² angenommen. Das bedeutet , daß das BMP-2 auf Grund eines etwa doppelt so großen "footprints" nur etwa die halbe Anzahl der Moleküle absorbiert werden, dafür auch etwa doppelt so viele immobilisierte Alkylreste (20-50) bedecken kann, von denen auch wiederum nach Berechnung der Erfinder vermutlich nur maximal 8-20 für Wechselwirkungen mit dem BMP-2 sterisch zur Verfügung stehen.

Von Versuchen der Erfinder mit Hexylagarosen, die nur etwa 7-8 Alkylreste/10 nm² besitzen, ist diesen bekannt, daß eine Adsorption ,von BMP-2 bei dieser geringen Anzahl von Wechselwirkungspartnern nicht möglich ist. Daher haben Versuche der Erfinder ergeben, daß nur in einem höheren Bereich von ca. 10-60 Alkylresten/10 nm² bei einem berechneten Abstand der Reste von 0.5-3 nm eine zufriedenstellende Adsorption von BMP-2 möglich ist. Eine Adsorption mit einer Halbwertszeit der Freisetzung von 90-100 Tagen ist nach Erkenntnis der Erfinder nur möglich, wenn eine Anzahl von mindestens 8-15 Alkylreste pro BMP-2-Molekül zur Reaktion an spezifischen Stellen zur Verfügung stehen kann. Diese Wechselwirkung wird allerdings nach Berechnung der Erfinder bei einem Substitutionsgrad von unter 10 Alkylresten/10 nm² statistisch wohl nur schlecht sein, so daß höhere Substitutionsgrade erfolgversprechender sind.

Seitens der Erfinder wurde festgestellt, daß eine Abhängigkeit der Kettenlänge der eingesetzten Alkylreste und des Abstandes der Alkylreste voneinander zur bestmöglichen Absorption von biologisch aktiven Knochenwachstumfaktoren als Mediatormoleküle besteht. Auf der einen Seite darf die Länge der Ketten nicht so groß sein, daß die Reste auf der Implantatoberfläche zusammengeknäuelt sind, auf der anderen Seite muß der Abstand der Reste zueinander so groß sein, daß diese nicht miteinander in Wechselwirkung treten. Je nach Größe des absorbierten Mediatormoleküles ergeben sich somit für den Einzelfall bestmögliche Werte für die Belegung der Oberfläche des Implantates hinsichtlich der Kettenlänge, des Verzweigungsgrades der Kette als auch des Abstandes der Reste. Für die Absorption des BMP-2 haben die Erfinder eine Belegung von 10 bis 60 Resten pro 10 nm², bevorzugt 10 bis 30 Resten pro 10 nm² bei einer Kettenlänge zwischen 1 bis 30, bevorzugt 1 bis 20, besonders bevorzugt 1 bis 8 Kohlenstoffatomen, bevorzugt in einer Kette, die auch gegebenenfalls mit einem oder mehreren Substituenten von Halogen, Alkoxy-, Hydroxyl-, Thiol-, Amino-, Alkyl- oder Dialkylamino-Gruppen substituiert sein können, festgestellt.

In einer bevorzugten Variante wird zur Erhöhung der Wechselwirkung zwischen den Resten auf der Oberfläche des Implantates und den Mediatormolekülen zunächst die Oberfläche des Implantates durch Aufbringen einer hydrophilen Beschichtung hydrophilisiert, in einem weiteren Schritt werden dann die hydrophoben Reste auf der so modifizierten Oberfläche immobilisiert und dann die Mediatormoleküle zur nichtkovalenten hydrophoben Wechselwirkung mit den Resten auf die Oberfläche gegeben, wobei für BMP-2 bevorzugt die oben angegebenen Verhältnisse von Kettenlänge und Belegungsgrad verwendet werden.

Das erfindungsgemäße Verfahren zur Immobilisierung der biologisch aktiven Knochenwachstumfaktoren als Mediatormoleküle zeichnet sich dadurch aus, dass das eingesetzte Implantatmaterial aus metallischen Materialien wie Reintitan oder metallischen Titanlegierungen wie Chrom/Nickel/Aluminium/Vanadium/Kobalt-Legierungen (z.B. TiAlV4, TiAlFe2,5), Edelstähle (z.B. V2A, V4A, Chrom-Nickel 316L) oder keramischen Materialien wie Hydroxylapatit, Aluminiumoxid oder aus einer Kombination, bei der z.B. metallisches mit keramischem Material beschichtet ist, besteht. Es sind auch Kunststoffpolymermaterialien zur Verwendung als Implantatmaterial geeignet.

Es ist auch Gegenstand der Erfindung, durch Beschichtung einer koronaren Gefäßstütze (sogenannter Koronarer Stent, Länge ca. 10 mm) mit Hilfe eines Biomoleküls oder eines Mediators, z.B. BMP-2, die Spätkomplikation Restenose, die durch eine Proliferation von glatten Gefäßmuskelzellen hervorgerufen wird, therapeutisch zu verhindern oder zu mildern, um damit die Einheilung und Verträglichkeit zu fördern.

Erfindungsgemäß können die Mediatormoleküle Biomoleküle sein, die vorteilhaft für die Biokompatibilität des Implantates sind, indem sie einer möglichen Abstoßung des Implantates entgegenwirken und/oder das Einwachsen des Implantates fördern.

Als Mediatormoleküle werden im vorliegenden Verfahren biologisch aktive Knochenwachstumsfaktoren eingesetzt, bevorzugt das Knochenwachstum fördernde Proteine aus der Klasse der Knochenwachstumsfaktoren 'Bone Morphogenic Proteins' oder auch Ubiquitin verwendet. Vorteilhaft kann zur Immobilisierung ein Protein dieser Klasse allein, in Kombination mit weiteren Mitgliedern dieser Klasse oder auch zusammen mit Biomolekülen wie Proteinen anderer Klassen oder niedermolekularen Hormonen oder auch Antibiotika zur Verbesserung der Immunabwehr eingesetzt werden. Dabei können diese Moleküle auch über im biologischen Milieu spaltbare Bindungen auf der Oberfläche Immobilisiert werden.

Erfindungsgemäß wird die Oberfläche des Implantatmaterials bevorzugt chemisch aktiviert, wobei die Aktivierung über ein Silanderivat wie beispielsweise γ-Aminopropyltriethoxysilan oder ein Trimethylmethoxy- bzw. Trimethylchlorsilanderivat oder 3-Glycidoxypropyltrimethoxysilan erfolgt und die Reaktion sowohl in einem wäßrigen als auch in einem organischen Lösungsmittel durchgeführt wird. An die so aktivierte Oberfläche kann in einem zweiten Schritt biologisch aktiven Knochenwachstumfaktoren als Mediatormoleküle mittels nicht-kovalenter Bindung auf dem Implantatmaterial immobilisiert werden.

Das Verfahren ist dadurch gekennzeichnet, daß man für die hydrophobe Interaktion als stationäre unlösliche Phase einen Träger verwendet, auf der an daran befindlichen gitterförmig angeordneten apolaren Gruppen eine monomolekulare, entropisch geordnete Wasserstruktur entsteht. Eine gleichartige geordnete monomolekulare Wasserschicht ist auf den hydrophoben Arealen des Proteins (BMP) vorhanden. Treten die beiden Moleküle (z.B. Alkylreste und BMP-2) mit ihren monomolekularen Wasserschichten miteinander in Kontakt, werden die Wasserschichten zerstört, indem aus der geordneten Wasserstruktur ein ungeordneteres System aus einzelnen Wassermolekülen wird. Die freie Energie der Wechselwirkung entsteht somit durch eine Zunahme der Entropie der Wassermoleküle. Bei höherer Temperatur werden diese hydrophoben Wechselwirkungen stärker (größere freie Energie).

Das BMP muß zur hydrophoben Interaktion mit einem geeigneten hydrophoben Träger gebracht werden. Ein solcher Träger besteht beispielsweise aus einer unlöslichen Phase und daran befindlichen hydrophilen und hydrophoben chemischen Strukturen. Insbesondere eignen sich als Träger alle festen Phasen mit hydrophilen Oberflächen, die zusätzliche hydrophobe/apolare Gruppen tragen.

Spezielle Beispiele für solche Träger organischer und anorganischer Art sind Zellulosen, Agarosen oder entsprechende mit Kohlenhydraten oder Polyhydroxykohlenstoffketten d. h. hydrophil beschichtete Polymerpartikel sowie, Silika-, Zeolit- oder Aluminiumhydroxidpartikel.

Neuartige Träger sind hydrophile Metalloberflächen, die beispielsweise in einem späteren Verfahren entsprechend mit Alkyl- oder Arylgruppen gitterförmig belegt/substituiert werden. Auf solchen festen Phasen liegen geeignete Substitutionsgrade mit hydrophoben Gruppen in einem Bereich von 0.01 bis 3.0 nmol/cm²' bevorzugt in einem Bereich von 0,01 bis 2,0 nmol/cm^{2.}, wobei die oben angegebenen Verhältnisse insbesondere bei Verwendung von BMP-2 eingehalten werden sollen.

Als hydrophile feste Phase eignen sich zur Substitution mit hydrophoben Gruppen vorzüglich in verdünnter Säure gereinigte Metalloberflächen oder mit Chromschwefelsäure veredelte Metalloberflächen mit Kontaktwinkeln zwischen 0-90°, vorzugsweise 0-20°. Insbesondere eignen sich als hydrophob interagierende mit verdünnter Säure gereinigte Metalloberflächen wie Titan, Stahl, Stahllegierungen wie Cr-Mo-Stahl oder mit Chromschwefelsäure veredelte Stahl- oder Titanoberflächen, die mit Methyl-, Ethyl-, Propyl-, Butyl-, Pentyl-, Hexyl-, Octyl-, Decyl-, Dodecyl-, oder Hexadecylgruppen substituiert wurde (Kettenlänge 1-30, bevorzugt 1-20, besonders bevorzugt 1 bis 8 Kohlenstoffatome, bevorzugt in einer Kette, die auch durch ein oder mehrere Substituenten wie Methyl-, Ethyl-, Methoxy-, Ethoxy-Gruppen oder Halogenatome wie Chlor, Fluor substituiert sein kann). Die hydrophobe Alkylwechselwirkung kann verstärkt werden durch Kombination mit einem Schwefelatom beispielsweise in Form einer Thioetherbindung oder als Thiol wie (Mercaptopropylreste).

Besondere Formen hydrophober Wechselwirkung können auch mit immobilisierten aromatischen Resten (Phenyl- oder Tolylreste, 6-7 C-Atome) insbesondere in Kombination mit Schwefelatomen (Phenylthiosilan, oder Thienylreste, 4-6 C-Atome) erzielt werden.

Die hydrophobe Interaktion an den Kontaktstellen erfolgt bei Temperaturen von 0° -100° C vorzugsweise bei 5-50° C bei einem pH-Wert von 3.0-11.0, vorzugsweise bei pH 6-10. Vorzugsweise geeignete Substitutionsgrade mit den Resten liegen bei 0.1-2,5 nmol/cm², was einem Gitterabstand der auf der Oberfläche kovalent gekoppelten Alkyl- oder Arylgruppen von 0.2-5 nm entspricht, bevorzugt 0.3-1 nm.

Bei einem Molekül von 5-6 nm Durchmesser, beispielsweise des BMP-2, könnte bei hohen Substitutionsgraden und somit kleinen Gitterabständen (0,2-1 nm) mehrere solcher Reste mit dem Molekül reagieren und es fest binden. Die Bindungsstärke (Affinität) der Oberfläche ist somit der Kettenlänge des Alkylrestes und dem Substitutionsgrad proportional und nimmt mit diesen Parametern stark zu. Eine vorzugsweise Bindung des BMP-2 erfolgt ab einer Kettenlänge von C-1, bevorzugt C-3 (Propyl) bei einem Substitutionsgrad von 0.01-2,5 nmol/cm², bevorzugt von 0.2-1 nmol/cm². Bei kürzeren Alkylketten sind höhere und bei längeren Ketten niedrigere Substitutionsgrade als Mindestgrößen bevorzugt.

Für die Synthese von alkyl- oder arylbeladenen Metalloberflächen eignen sich Alkyltrichlorsilane (Methyltrichlorosilan, Ethyltrichlorosilan, Propyltrichlorosilan, etc.), Dialkyldichlorsilane (Dimethyldichlorosilan, Diethyldichlorsilan, Dipropyldichlorsilan etc. ), Trialkylchlorosilane (Trimethylchlorosilan, Ethyldimethylchlorosilan, Propyl. etc.), Alkyltrimethoxysilane Methyltrimethoxysilan, Ethyl-, Propyl- etc.), Alkyltriethoxysilane (Methyltriethoxysilan, Ethyl-, Propyl- etc.), Phenyltrichlorosilan, Phenyldimethylchlorosilan, Phenylthiotrimethylsilan, p-Tolyltrichlorosilan.

Weitergehende Untersuchungen der Erfinder haben gezeigt, dass die Verankerung der Alkyl- oder Arylreste auf der Oberfläche des Implantatmaterials qualitativ und quantitativ verbessert werden kann, indem man auf der Implantatoberfläche eine hydrophile Beschichtung, beispielsweise Agarose, Polyacrylat, oder vorzugsweise durch Erhöhung der Anzahl der auf der Oberfläche verfügbaren Metalloxid-Einheiten vorsieht.

Seitens der Erfinder wurde gefunden, dass die Anzahl der Oxidgruppen überraschenderweise dadurch erhöht werden kann, dass die Oberfläche des Metalls mit heißer, vorzugsweise bodensatzfreier, Chromschwefelsäure behandelt wird. Im Gegensatz zu der Erwartung, dass sich das Metall unter diesen Bedingungen auflöst, wird bei Verwendung dieser Säure eine neuartige im wesentlichen gleichmäßige 5-50 nm dicke hydrophile Oxidschicht auf der Oberfläche des Metalls erzeugt. Das Verfahren ist so schonend, daß selbst koronare Gefäßstützen, sogenannte Stents (die z.B. aus Edelstahl oder Titan gefertigt sein können) ohne Zerstörung des dünnen empfindlichen Gitterwerkes (50-150 µm Durchmesser) beschichtet werden können. Bei Großimplantaten kann die hydrophile Oxidschicht unter definierten Bedingungen eine Dicke von 10 µm bis zu 100 µm erreichen und relativ "glatt" ohne Vertiefungen oder Löcher aufgebaut werden. Als Metall für das Implantat kann dabei Reintitan oder Titanlegierungen (z.B. TiAlV4, TiAlFe2,5), Aluminium oder rostfreier Stahl (z.B. V2A, V4A, Chrom-Nickel 316L, Cr-Mo-Stahl) eingesetzt werden. Eine handelsübliche Chromschwefelsäure mit 92 Gew.-% H₂SO₄, 1,3 Gew.-% CrO₃ und einer Dichte von 1,8 g/cm³ beispielweise von der Firma Merck erhältlich, wird bevorzugt zur Erzielung einer dünnen glatten Schicht aus Metalloxid verwendet. Dazu wird das Metallsubstrat in die Chromschwefelsäure eingelegt und über einen Zeitraum von 1 bis zu 3 Stunden bei 100 bis 250°C behandelt, vorzugsweise 30 bis 60 Minuten bei 240°C danach sorgfältig mit Wasser abgespült, danach in Wasser oder einer Lösung von 1-4 % EDTA (Ethylendiamintetraacetat) pH 7,0, vorzugsweise 2% EDTA pH 7,0 30 min gekocht, um auf der Oberfläche verbliebene Schwermetallionen, z.B. Chromionen zu entfernen, und dann getrocknet.

Wenn eine dickere Metalloxidschicht an der Metalloberfläche und/oder bevorzugt eine Oxidschicht mit kleinen Mikro- und Nanoporen versehen werden soll, wird die oben beschriebene Chromschwefelsäure mit Wasser auf eine Dichte von 1,5 bis 1,6 g/cm³ verdünnt. Bei einer dann folgenden wie oben beschriebenen Behandlung des Metallimplantatoberfläche mit der so verdünnten Säure wird eine "rauhe" Oberflächenschicht mit Vertiefungen und Poren ausgebildet, so dass die zur Beladung mit Mediatormolekülen zur Verfügung stehende Oberfläche vergrößert wird. Durch Einstellung unterschiedlicher Dichten an Chromschwefelsäure und unterschiedliche Behandlungszeiten und -temperaturen ist es daher möglich, eine Vielzahl verschiedener Oxidschichten unterschiedlicher Eigenschaften auf Metalloberflächen mit hoher Haftfestigkeit aufzubringen. Die Erfindung ist daher auch auf ein solches Verfahren zur Ausbildung einer thermodynamisch einheitlichen Metalloxidschicht (keine Kontaktwinkelhysterese) auf dem Implantatmaterial mittels heißer Chromschwefelsäure gerichtet.

Die Metalloxidschicht auf dem Implantatmaterial aus den oben genannten Materialien kann dann über Behandlung mit verdünnter Salpetersäure (ca. 5 Gew.-%) und anschließende Kopplung eines Silanderivates, aktiviert werden.

Über die Moleküle des Silanderivates können dann die Mediatormoleküle auf der Implantatoberfläche nicht-kovalent verankert werden.

Als Implantatmaterial kann auch ein keramisches Material wie beispielsweise Hydroxylapatit verwendet werden. Das Hydroxylapatit sollte dabei zunächst durch Behandlung mit Aminoalkylsilan aktiviert werden und die Ankermoleküle dann verankert werden. Erfindungsgemäß sind unter Ankermoleküle solche Moleküle zu verstehen, die auf der Oberfläche des Implantates verankert werden und mit den Mediatormolekülen nicht-kovalent Wechselwirkungen zeigen, wenn im nächsten Schritt eine nicht-kovalente Bindung der Mediatormoleküle wie BMP an die Oberfläche erfolgt.

Falls unter den Kopplungsbedingungen die eingesetzten Mediatoren im Medium schwerlöslich sind, kann die Löslichkeit durch Zugabe von Tensiden/Detergentien erhöht und die Reaktion durchgeführt werden. So können bei pH-Werten > 6 schwerlösliche Knochenwachstumsfaktoren und andere Mediatoren durch ionische oder nichtionische Detergentien im Konzentrationsbereich 0.05-10%, vorzugsweise 1 -5 Gew.%, insbesondere bei 0.066% SDS bei pH-Werten > 6, insbesondere bei pH 8-10 für nicht-kovalente Bindungsverfahren im alkalischen pH-Bereich ohne Verlust der biologischen Aktivität in Lösung gehalten werden.

Der Einfluß der nach dem erfindungsgemäßen Verfahren modifizierten Materialien auf Knochenzellen wurde im Tierversuch untersucht, wobei die modifizierten Materialien zu diesem Zweck in Plättchen- oder Hantelform hergestellt wurden. Dabei wurde beobachtet, dass es 4 Wochen nach dem Einbringen in die Tiere zu einer beschleunigten Knochenbildung mit Kontakt zur Implantatoberfläche durch BMP-2 auf den Materialien kam.

Anhand der folgenden Beispiele wird die vorliegende Erfindung weiter veranschaulicht.

### Modifikation von Metallen (Titan, 316 L rostfreier Stahl):

Es können entweder mechanisch polierte/elektropolierte, anodisch oxidierte Titanplättchen oder mit poröser Titanlegierung plasmagespritzte Titanlegierungsplättchen mit oder ohne Chromschwefelsäureveredelung eingesetzt werden. Gleichermaßen können rostfreie mechanisch polierte/elektropolierte Stähle mit oder ohne Chromschwefelsäureveredelung eingesetzt werden.

### Reinigungsverfahren

Vor jedem Einsatz werden die Metalle gereinigt durch Erhitzen auf 80 °C in 5% HNO₃ für 2 Stunden. Nach erneutem Waschen in Wasser wurden die Plättchen durch Waschen in 30 ml trockenem Methanol getrocknet. Danach wurden sie entweder direkt weiter verwendet oder mit Chromschwefelsäure veredelt.

### Chromschwefelsäureveredelung

Bei der Chromschwefelsäureveredelung wurden die Titanplättchen bei 190-240 °C für 30-90 min und die Stahlplättchen bei 190-230 °C für 30-90 min in Chromschwefelsäure (92% H₂SO₄, 1.35 CrO₃) inkubiert. Danach wurden die Metallproben mit Wasser ausgiebig gewaschen und dann in 2% EDTA pH 7.0 und anschließend in Wasser für jeweils 30 Minuten gekocht. Nach erneutem Waschen in Wasser wurden die Plättchen durch Waschen in 30 ml trockenem Methanol getrocknet.

### Beladung von Oberflächen mit Aminopropyltriethoxysilan:

Die gereinigten Träger (5-10 Titanplättchen) wurden mit oder ohne Chromschwefelsäureveredelung in einem Teflonhalter mit 47.5 ml Toluol und 2.5 ml Aminopropylthriethoxysilan unter Inertgas versetzt und verschlossen. Sodann wurde der Ansatz unter Rückfluß und unter langsamem Rühren 3-3.5 Stunden gekocht. Die Plättchen wurden dann 3 mal mit 10 ml Trichlormethan, Aceton und Methanol gewaschen und dann luftgetrocknet. Bei der angegebenen Aminopropyltriethoxysilankonzentration konnten mit Hilfe der Bolton-Hunter-Methode eine Oberflächenkonzentration an Aminogruppen von 1.5 bis 2.5 nmol/cm² bestimmt werden.

### Beladung von Oberflächen mit Trialkylmonochlorsilanen:

Die gereinigten Träger (Metallplättchen) wurden mit oder ohne Chromschwefelsäureveredelung mit einer 5%igen Trialkylsilanlösung (v/V) in trockenem Toluol versetzt, die zusätzlich 5% Pyridin (v/v) enthält. Nach einer Reaktionsdauer von 1-3 Std. wird mit Ethanol, 0.01M Salzsäure und dest. Wasser gewaschen. Bei bedarf können die Träger bei 60 - 110 °C im Vakuum getrocknet werden.

### Beladung von Oberflächen mit Alkyltrimethoxysilanen:

Die gereinigten Träger (Metallplättchen) wurden mit oder ohne Chromschwefelsäureveredelung mit einer 5%-igen Lösung Alkyltrimethoxysilanlösung (v/V) in trockenem Trichloroethylen versetzt. Nach einer Reaktionsdauer von 12 Std. bei Raumtemperatur wird mit Trichlorethylen, Aceton und Ethanol gewaschen. Im Falle von Mercaptopropyltrimethoxysilan muß UV-Licht ausgeschlossen werden. Bei Bedarf können die Träger (ohne SH-Gruppen) bei 100-110° im Vakuum getrocknet werden.

### Beladung von Oberflächen mit Dichlordialkyl- und

### Trichloralkylsilanen:

Die gereinigten Träger (Metallplättchen) wurden mit oder ohne Chromschwefelsäureveredelung wurden mit einer 5-10%igen Dichlordialkyl- oder Trichloralkylsilanlösung (v/V) in trockenem Toluol versetzt. Nach einer Reaktionsdauer von 1-3 Std. wird mit Ethanol und dest. Wasser gewaschen. Bei Bedarf können die Träger bei 60-110 °C im Vakuum getrocknet werden.

### Bindung von rhBMP-2 an ein Propylamin-Titanbindungsgitter:

Die Propylamin-beschichteten Titanplättchen wurden mit 125 mM NaBorat Puffer, 0.066% Natriumdodecylsulfat, pH 10.0 gewaschen und äquilibriert. Sie wurden dann mit einer rhBMP-2 Lösung (rekombinantes humanes BMP-2) (0.2-0.3 mg/ml in 125 mM NaBorat Puffer, 0.066% Natriumdodecylsulfat, pH 10.0) versetzt und 12-14 Stunden bei Raumtemperatur unter Schütteln inkubiert. Danach wurden sie 4 x mit Boratpuffer gewaschen und anschließend mit Wasser. Bindung rhBMP-2 an elektropoliertes Titan: 10-30ng/cm² Bindung rhBMP-2 an chromschwefelsäureveredeltes Titan: 2-10ng/cm² Bindung rhBMP-2 an chromschwefelsäureveredeltes Propylamin-Titanbindungsgitter: 100-270ng/cm²

Ähnlich hohe Werte lassen sich bei einem reinen Propyl-Titanbindungsgitter auch für chromschwefelsäureveredeltes Titan erzielen. Dabei ist zu beobachten, dass das hydrophob adsorbierte BMP-2 sich allerdings nicht durch ausgedehntes Waschen mit Pufferlösungen oder Wasser abwaschen läßt.

Wie oben angegeben, konnte erstaunlicherweise die nicht-kovalent gebundene Beladung mit BMP-2 auch nicht durch Verwendung eines Tensids wie mit einer 1% SDS-Lösung entfernt werden, was auf äußerst starke Adsorptionskräfte schließen läßt. Diese hydrophoben Wechselwirkungen können durch Charge-Transfer-Komplexe, H-Brückenbildung und Ladungsschwächung verstärkt werden, während eine Substitution der Kette mit Hydroxyl- oder Thiol-Gruppen sowie eine Ladungsverstärkung durch z.B. Ammoniumreste zur Schwächung der hydrophoben Wechselwirkungen führt.

Dabei stellten die Erfinder bei ihren Versuchen fest, daß eine kontrollierte Abgabe von BMP-2 durch eine an dem Alkylrest vorhandene positive Ladung entscheidend beeinflußt werden kann. Dabei spielt der pK-Wert der alkalischen Gruppe -NH₂ eine wichtige Rolle, der bei pK 8-12 liegen kann und durch eine Substitution des Stickstoffes, beispielsweise zum quarternären Ammoniumion, stark beeinflußt werden kann, so daß eine vom pH abhängige ladungsbeeinfußte Adsorption und spätere Freisetzung des BMP-2 an der Oberfläche erfolgen.

Auch bei einem pH-Wert von 7,0, im physiologischen Bereich, ist die nicht kovalente Bindung zwischen den auf dem Metall immobilisierten hydrophoben Liganden und dem BMP-2 außerordentlich stabil, so daß pro Tag maximal 0,1-1% des adsorbierten BMP's freigesetzt wird. Da im Fall von mit Aminogruppen substituierten Gruppen auf der Implantatoberfläche sowohl die Aminogruppen als auch das BMP bei einem pH-Wert von 7,0 positiv geladen sind, ist dabei eine elektrostatische Adsorption praktisch ausgeschlossen.

Die oben beschriebenen Versuche wurden unter entsprechend angepassten Bedingungen mit den übrigen in der beigefügten Tabelle Verbindungen durchgeführt. Dabei handelt sich um Mittelwerte von jeweils 4 Experimenten mit Standardabweichung. Die Plättchen (5 x 10 x 1 mm ; = 1 cm²) wurden nach Vorreinigung mit HNO₃ oder nach Vorbehandlung mit Chromschwefelsäure einzeln 4x in 125 mM Borat, 0.066% SDS, pH 10, 15 min gewaschen. Die Adsorptionsbedingungen waren wie folgt: ¹²⁵I-BMP-2-Lösung: C_{bmp} = 0.1mg/ml in 125 mM Borat, 0.066% SDS, pH 10; 12-14 Std. bei 5°C.

Die in der Tabelle verwendeten Abkürzungen haben die folgende Bedeutung:

| | |
|---|---|
| Ti-EP | elektropoliertes Metall |
| Ti-CSB | mit Chromschwefelsäure behandeltes Metall |
| θ_{V} | Vorrückwinkel (Randwinkelmessung nach Wilhelmy) |
| θ _{R} | Rückzugswinkel (Randwinkelmessung nach Wilhelmy) |
| t_{1/2} | Halbwertszeit der Freisetzung von ¹²⁵I-rhBMP-2 |

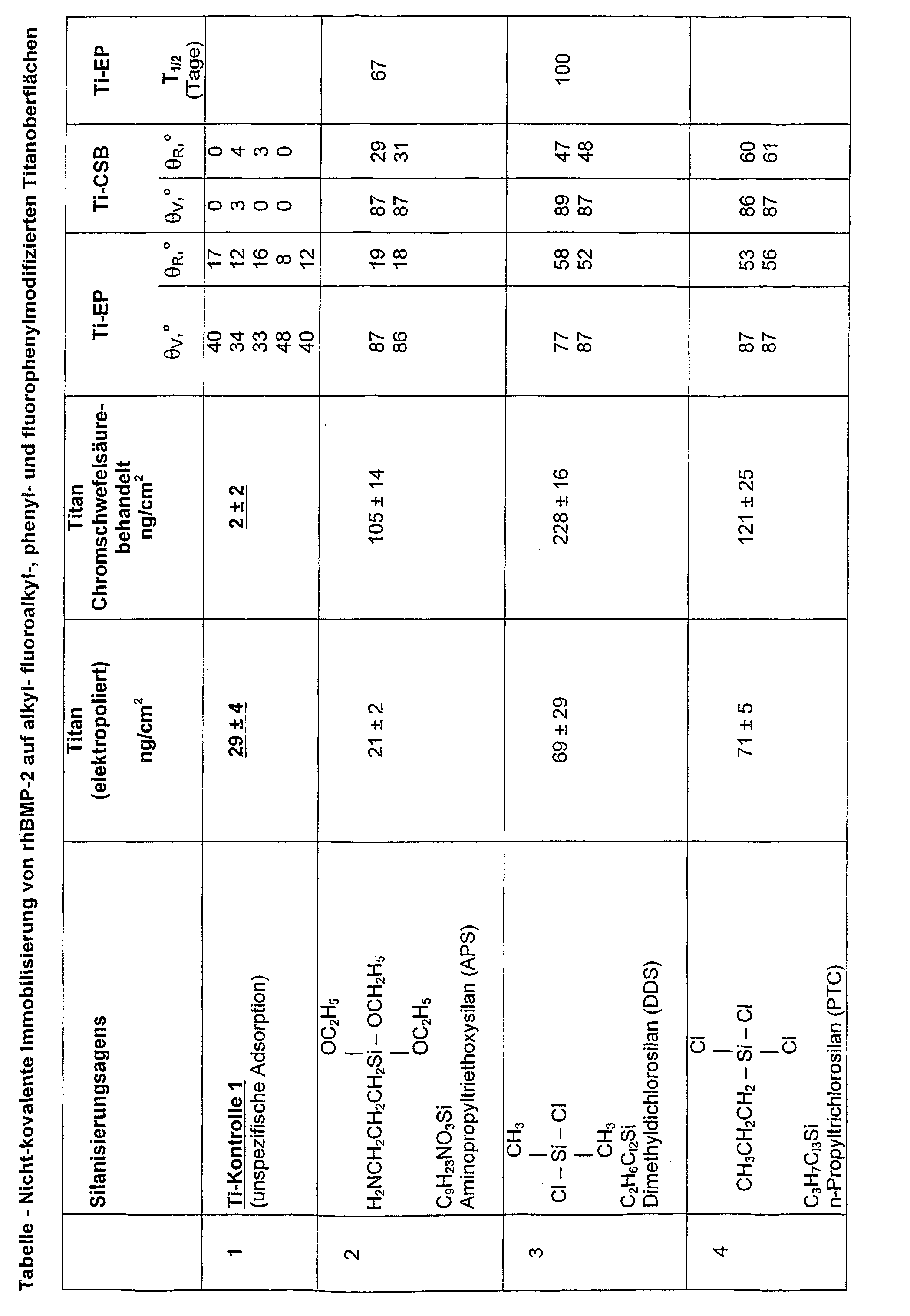

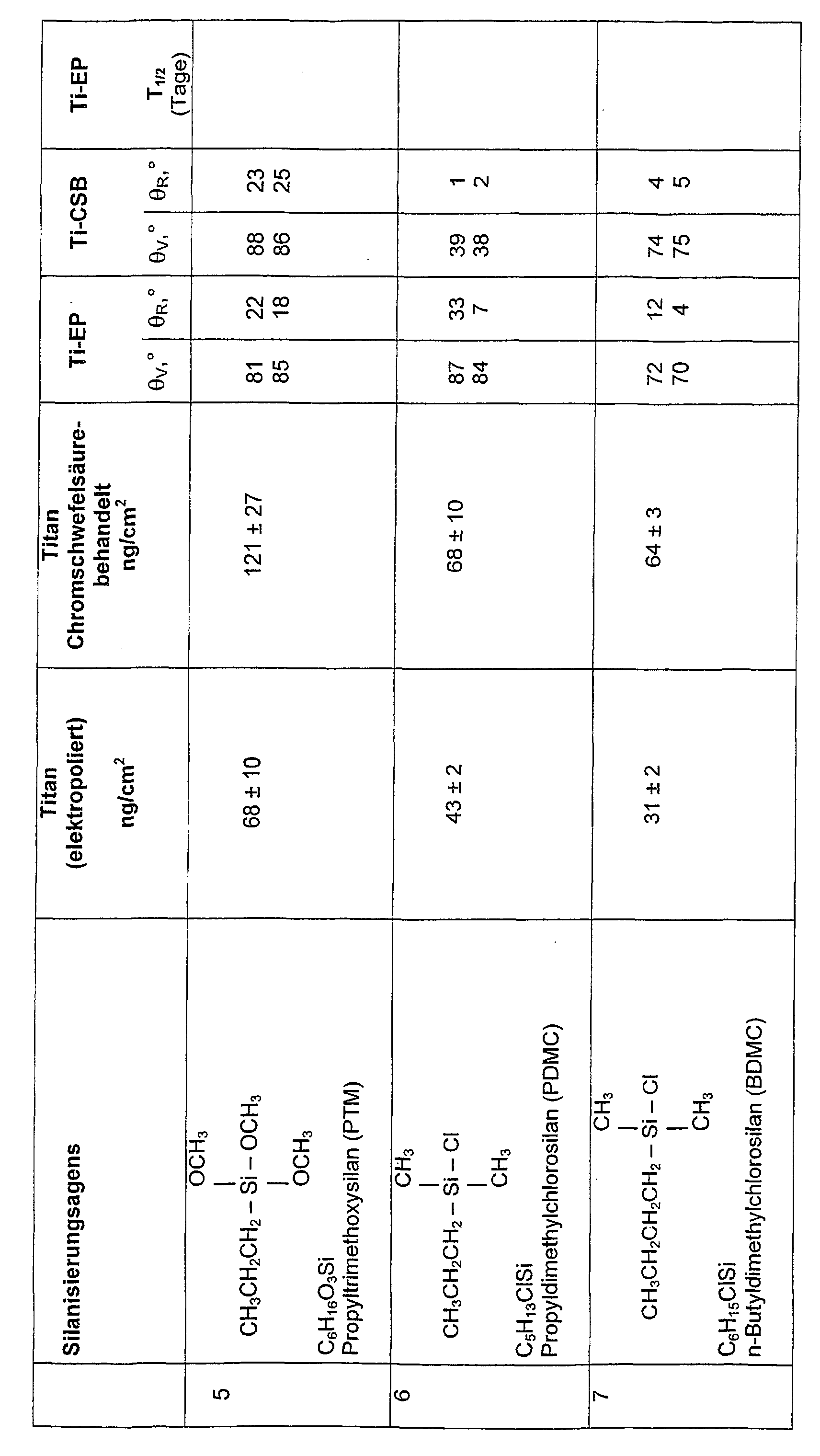

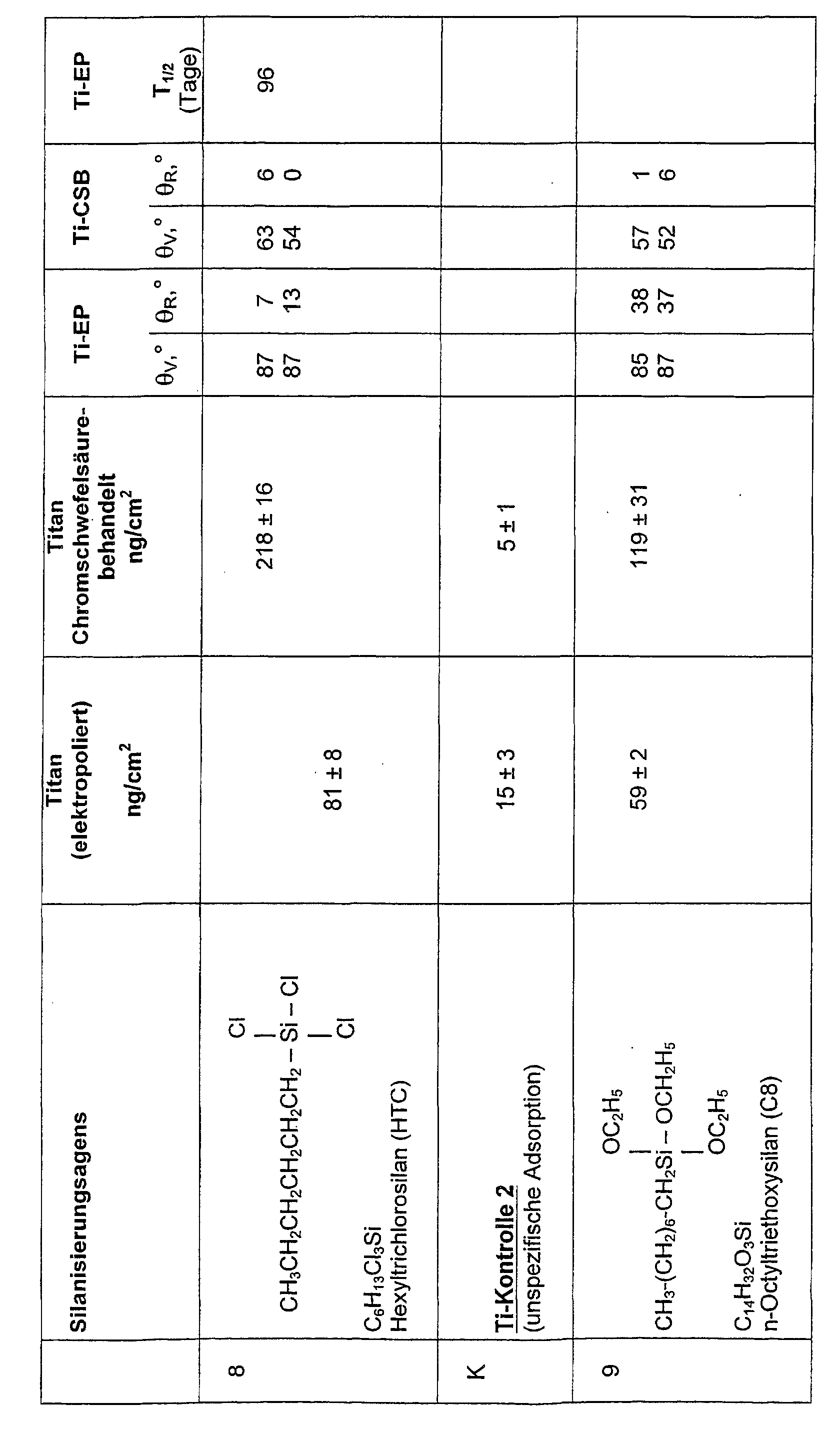

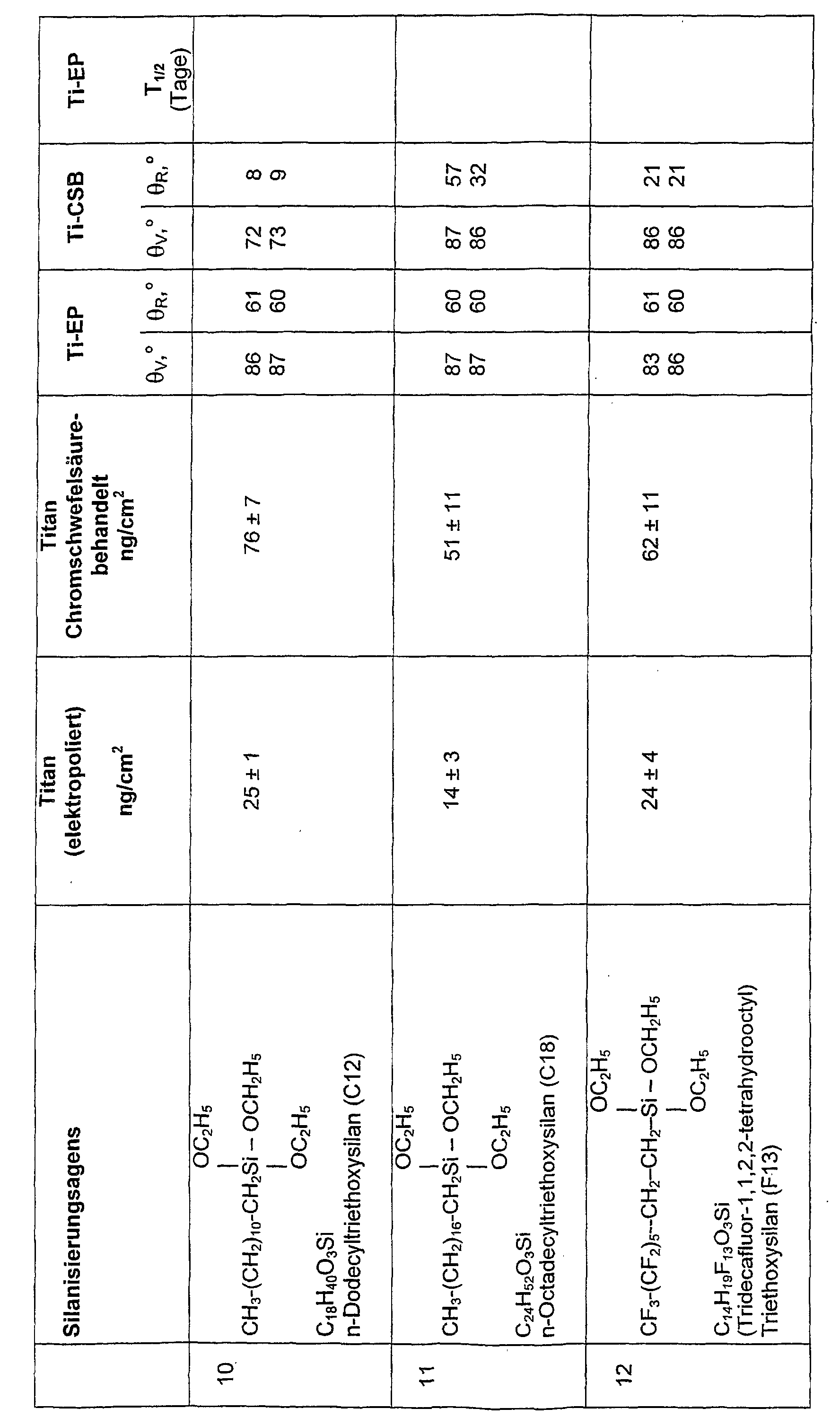

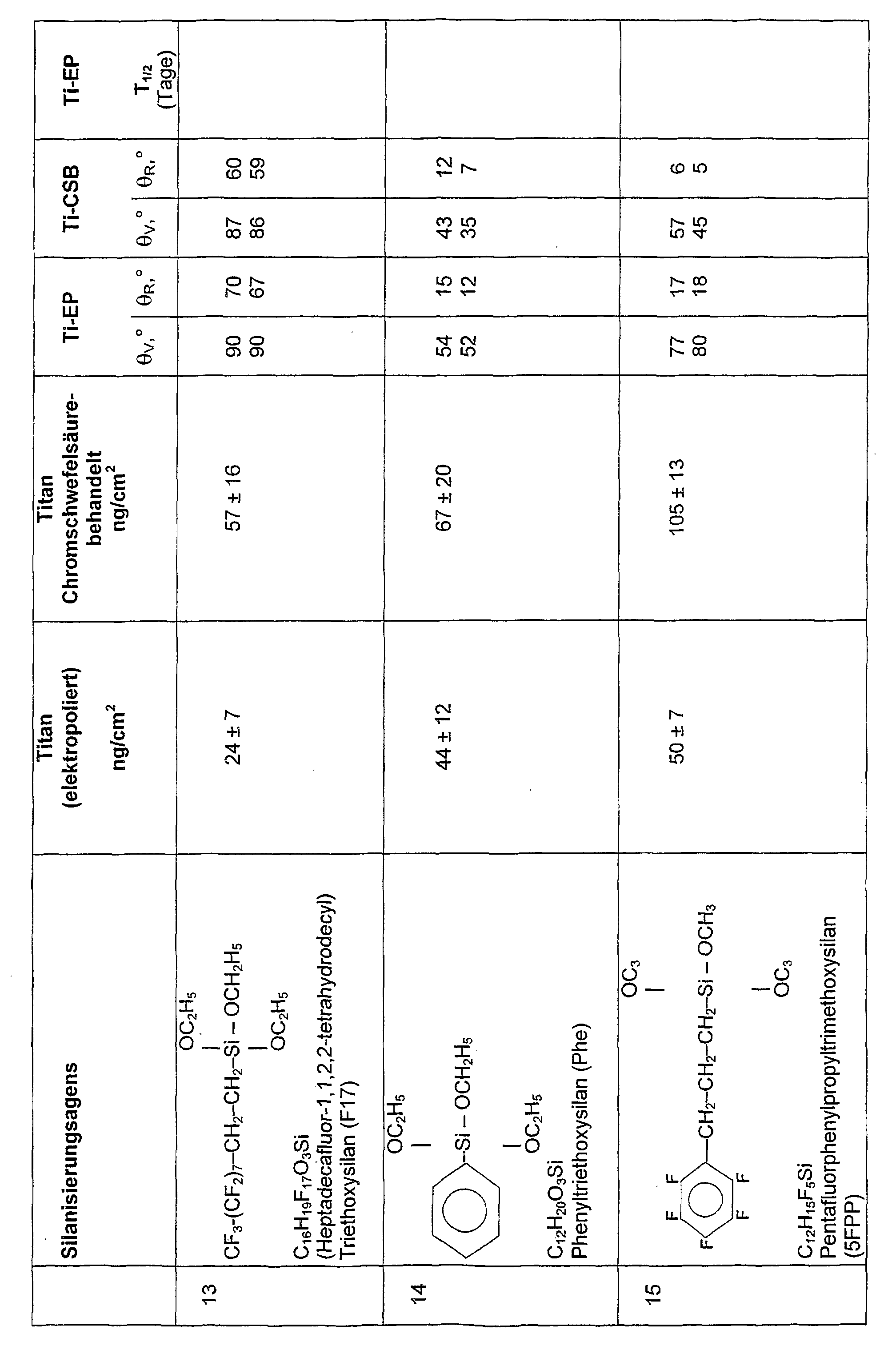

## Patentansprüche

1. Verfahren zur Herstellung bioaktiver Implantatoberflächen von metallischen oder keramischen Materialien,
a. bei dem in einem ersten Schritt Ankermoleküle mit hydrophoben Resten an der Oberfläche des Implantatmaterials kovalent gebunden werden und
b. in einem zweiten Schritt Mediatormoleküle, die infolge nicht-kovalenter Wechselwirkungen zwischen den Mediatormolekülen und den hydrophoben Resten der Ankermoleküle immobilisiert werden, auf das so behandelte Implantatmaterial gegeben werden,
wobei im ersten Schritt die Beladungsdichte der Ankermoleküle auf der Implantatoberfläche in Abhängigkeit von der Kettenlänge des hydrophoben Restes des Ankermoleküls so gewählt wird, daß die Ankermoleküle selbst miteinander nicht in Wechselwirkung treten und in Abhängigkeit von der überdeckten Fläche auf dem Implantatmaterial, die von einem einzelnen im zweiten Schritt absorbierten Mediatormolekül bedeckt wird, mindestens 10, bevorzugt 15 Kontaktstellen zwischen den hydrophoben Resten der Ankermoleküle zur hydrophoben Wechselwirkung mit dem einzelnen Mediatormolekül gebildet werden,
wobei die hydrophoben Reste der Ankermoleküle Reste mit 3 bis 20, bevorzugt 3 bis 8 Kohlenstoffatomen, die auch durch Silizium- oder Heteroatome wie N, O oder S in der Kette ersetzt sein können, sind, wobei die hydrophoben Reste auch gegebenenfalls mit einem oder mehreren Substituenten von Halogen, Alkoxy-, Hydroxyl-, Thiol-, Amino-, Alkylamino-, Dialkylamino oder Trialkylamino-Gruppen, wobei die Alkylgruppen des Substituenten bevorzugt 1 - 6 Kohlenstoffatome aufweisen und geradkettig oder verzweigt sein können, substituiert sein können, und
wobei als Mediatormoleküle biologisch aktive Knochenwachstumsfaktoren eingesetzt werden.

2. Verfahren nach Anspruch 1, wobei als biologisch aktive Knochenwachstumsfaktoren solche aus der Klasse der BMP-Proteine eingesetzt werden.

3. Verfahren nach einem der vorhergehenden Ansprüche, bei dem das Implantatmaterial aus einem Material, ausgewählt aus der Gruppe der Metalle, der metallischen Legierungen, der keramischen Materialien oder Kombinationen davon, besteht.

4. Verfahren nach einem der vorhergehenden Ansprüche, bei dem als Mediatormoleküle neben Knochenwachstumsfaktoren aus der Klasse der BMP-Proteine Antibiotika eingesetzt werden.

5. Verfahren nach einem der vorhergehenden Ansprüche, bei dem als Knochenwachstumsfaktor BMP-2 oder BMP-7 verwendet wird.

6. Verfahren nach einem der vorhergehenden Ansprüche, bei dem die Oberfläche des Implantatmaterials vor der kovalenten Bindung der Ankermoleküle mit einer hydrophilen Beschichtung versehen wird.

7. Verfahren nach Anspruch 6, bei dem die hydrophile Beschichtung eine hydrophile Oxidschicht ist.

8. Verfahren nach Anspruch 6 oder 7, bei dem die Oberfläche des Implantatmaterials, ausgewählt aus Titan, Titanlegierungen, Aluminium oder rostfreiem Stahl, vor der kovalenten Bindung der Ankermoleküle durch Behandlung mit Chromschwefelsäure über einen Zeitraum von 0,5 bis zu 3 Stunden bei 100 bis 250°C mit einer Oxidschicht versehen wird.

9. Verfahren nach Anspruch 8, bei dem die Chromschwefelsäure eine Dichte von mehr 1,40 g/cm³ hat.

10. Verfahren nach einem der vorhergehenden Ansprüche, bei dem die Kohlenstoffreste im ersten Schritt je nach Verzweigungsgrad des verwendeten Restes in einer Anzahl von mindestens 3, bevorzugt mindestens 5 und besonders bevorzugt mindestens 10 Resten auf 10 nm² der Implantatoberfläche immobilisiert werden.

11. Verfahren nach Anspruch 10, bei dem die Kohlenstoffreste im ersten Schritt je nach Verzweigungsgrad des verwendeten Restes in einer Anzahl von maximal 100, bevorzugt maximal 60 Resten auf 10 nm² der Implantatoberfläche immobilisiert werden.

12. Implantat, erhältlich nach dem Verfahren nach einem der Ansprüche 1-11.

13. Implantat nach Anspruch 12, bei dem das Implantatmaterial aus Titan, Titanlegierungen, Aluminium, rostfreiem Stahl, Stahlegierungen oder Hydroxylapatit besteht.

14. Implantat nach Anspruch 12 oder 13, bei dem das Implantat eine Gelenk- oder Knochenprothese, ein Stent oder ein Zahnimplantat ist.

## Claims

1. A process for the production of bioactive implant surfaces of metallic or ceramic materials,
a. in which, in a first step, anchor molecules having hydrophobic residues are covalently bonded to the surface of the implant material, and
b. in a second step, mediator molecules which, as a result of none-covalent interactions between the mediator molecules and the hydrophobic residues of the anchor molecules, are immobilized, are added to the implant material treated in this way,
where in the first step the loading density of the anchor molecules on the implant surface is chosen, depending on the chain length of the hydrophobic residue of the anchor molecule, such that the anchor molecules do not interact with one another themselves and, depending on the covered surface on the implant material, which is covered by an individual mediator molecule absorbed in the second step, at least 10, preferably 15 contact sites are formed between the hydrophobic residues of the anchor molecules for hydrophobic interaction with the individual mediator molecule, wherein the hydrophobic residues of the anchor molecules are residues with 3 to 20, preferably 3 to 8, carbon atoms, which can also be replaced by silicon or heteroatoms such as N, O or S in the chain, where the hydrophobic residues can also optionally be substituted by one or more substituents from halogen, alkoxy, hydroxyl, thiol, amino, alkyl-amino, dialkyl-amino or trialkyl-amino groups, where the alkyl groups of the substituent preferably have 1 - 6 carbon atoms and can be straight-chained or branched, and wherein biologically active bone growth factors are used as mediator molecules.

2. The process according to claim 1, wherein the used biologically active bone growth factors are those from the class of BMP proteins.

3. The process according to any of the preceding claims, in which the implant material consists of a material selected from the group consisting of the group of metals, metallic alloys, ceramic materials or combinations thereof.

4. The process according to any of the preceding claims, in which antibiotics are used as mediator molecules in addition to bone growth factors from the class of BMP proteins.

5. The process according to any of the preceding claims, in which BMP-2 or BMP-7 are used as bone growth factors.

6. The process according to any of the preceding claims, in which the surface of the implant material is provided with a hydrophilic coating before the covalent bonding of the anchor molecules.

7. The process according to claim 6, in which the hydrophilic coating is a hydrophilic oxide layer.

8. The process according to claim 6 or 7, in which the surface of the implant material, selected from titanium, titanium alloys, aluminium or stainless steel, is provided with an oxide layer before the covalent bonding of the anchor molecules by treatment with chromosulfuric acid for a period of 0.5 up to 3 hours at 100 to 250°C.

9. The process according to claim 8, in which the chromosulfuric acid has a density of more than 1.40 g/cm³.

10. The process according to any of the preceding claims, in which the carbon residues are immobilized in the first step, depending on the degree of branching of the residue used, in a number of at least 3, preferably at least 5 and particularly preferably at least 10 residues to 10 nm² of the implant surface.

11. The process according to claim 10, in which the carbon residues are immobilized in the first step, depending on the degree of branching of the residue used, in a number of at most 100, preferably at most 60 residues per 10 nm² of the implant surface.

12. An implant, obtainable by the process according to any of the claims 1 to 11.

13. The implant according to claim 12, in which the implant material consists of titanium, titanium alloys, aluminium, stainless steel, steel alloys or hydroxyapatite.

14. The implant according to claim 12 or 13, in which the implant is a joint or bone prosthesis, a stent or a dental implant.

## Revendications

1. Procédé de fabrication de surfaces d'implant bioactives en matériaux métalliques ou céramiques,
a. dans lequel, dans une première étape, des molécules d'ancrage avec résidus hydrophobes sont liées de façon covalente à la surface du matériau de l'implant, et
b. dans une deuxième étape, on dépose sur le matériau de l'implant ainsi traité des molécules médiatrices, qui sont immobilisées par suite des interactions non covalentes entre les molécules médiatrices et les résidus hydrophobes des molécules d'ancrage,
dans lequel, dans la première étape, la densité de charge des molécules d'ancrage sur la surface de l'implant est choisie en fonction de la longueur de chaîne du résidu hydrophobe de la molécule d'ancrage, de telle sorte que les molécules d'ancrage elles-mêmes n'entrent pas en interaction les unes avec les autres et, en fonction de la surface non recouverte sur le matériau de l'implant, qui est couverte par une seule molécule médiatrice absorbée au cours de la deuxième étape, il se forme au moins 10, de préférence 15 zones de contact entre les résidus hydrophobes des molécules d'ancrage pour l'interaction hydrophobe avec la seule molécule médiatrice,
dans lequel les résidus hydrophobes des molécules d'ancrage sont des résidus avec 3 à 20, de préférence 3 à 8 atomes de carbone, qui peuvent aussi être remplacés dans la chaîne par des atomes de silicium ou des hétéroatomes, tels que N, O ou S, les résidus hydrophobes pouvant aussi, le cas échéant, être substitués par un ou plusieurs substituants d'halogène, de groupes alkoxy, hydroxyl, thiol, amino, alkylamino, dialkylamino ou trialkylamino, les groupes alkyl des substituants comportant de préférence 1 à 6 atomes de carbone et pouvant être en chaîne linéaire ou ramifiée, et
dans lequel les molécules médiatrices utilisées sont des facteurs de croissance osseuse biologiquement actifs.

2. Procédé selon la revendication 1, dans lequel les facteurs de croissance osseuse biologiquement actifs utilisés sont ceux issus de la classe des protéines BMP.

3. Procédé selon l'une quelconque des revendications précédentes, dans lequel le matériau de l'implant est composé d'un matériau choisi dans le groupe des métaux, des alliages métalliques, des matériaux céramiques ou des combinaisons de ceux-ci.

4. Procédé selon l'une quelconque des revendications précédentes, dans lequel, outre les facteurs de croissance osseuse biologiquement actifs issus de la classe des protéines BMP, les molécules médiatrices utilisées sont des antibiotiques.

5. Procédé selon l'une quelconque des revendications précédentes, dans lequel le facteur de croissance osseuse utilisé est une BMP-2 ou BMP-7.

6. Procédé selon l'une quelconque des revendications précédentes, dans lequel la surface du matériau de l'implant est recouverte par un revêtement hydrophile, avant la liaison covalente des molécules d'ancrage.

7. Procédé selon la revendication 6, dans lequel le revêtement hydrophile est une couche d'oxyde hydrophile.

8. Procédé selon la revendication 6 ou 7, dans lequel, avant la liaison covalente des molécules d'ancrage, la surface du matériau de l'implant, choisie en titane, alliages de titane, aluminium ou acier inoxydable, est revêtue d'une couche d'oxyde par un traitement à l'acide sulfochromique pendant un intervalle de temps de 0,5 à 3 heures à 100 à 250°C.

9. Procédé selon la revendication 8, dans lequel l'acide sulfochromique a une masse volumique supérieure à 1,40 g/cm³.

10. Procédé selon l'une quelconque des revendications précédentes, dans lequel les résidus de carbone dans la première étape, en fonction du degré de ramification du résidu utilisé, au nombre d'au moins 3, de préférence d'au moins 5 et de manière particulièrement préférée d'au moins 10 résidus, sont immobilisés sur 10 nm² de la surface de l'implant.

11. Procédé selon la revendication 10, dans lequel les résidus de carbone dans la première étape, en fonction du degré de ramification du résidu utilisé, au nombre de 100 maximum, de préférence 60 résidus maximum, sont immobilisés sur 10 nm² de la surface de l'implant.

12. Implant obtenu par le procédé selon l'une quelconque des revendications 1 à 11.

13. Implant selon la revendication 12, dans lequel le matériau de l'implant est composé de titane, d'alliages de titane, d'aluminium, d'acier inoxydable, d'alliages d'acier ou d'hydroxylapatite.

14. Implant selon la revendication 12 ou 13, dans lequel l'implant est une prothèse articulaire ou osseuse, un stent ou un implant dentaire.
